# EUROPEAN PATENT APPLICATION

(11) **EP 4 851 146 A1**
(43) Date of publication of application: **09.12.2026**
(21) Application number: 25747805.7
(22) Date of filing: 24.01.2025
(51) Int. Cl.: C07D 401/12, A61P 25/22, A61P 25/24

(54) **SALT FORM AND CRYSTAL FORM OF 5-HT2A RECEPTOR INVERSE AGONIST, PREPARATION METHOD THEREFOR AND USE THEREOF**

(30) Priority: 02.02.2024 CN 202410148990
(71) Applicant: Luye Innomind Pharma Shijiazhuang Co., Ltd., Shijiazhuang, Hebei 050000 (CN)
(72) Inventor: GAO, Shuai, Yantai, Shandong 264670 (CN); WANG, Guanghui, Yantai, Shandong 264670 (CN); SHI, Zuopan, Yantai, Shandong 264670 (CN); QU, Hao, Yantai, Shandong 264670 (CN); WU, Yanhua, Yantai, Shandong 264670 (CN); LIAO, Wensheng, Yantai, Shandong 264670 (CN)
(74) Representative: karo IP
(86) International application number: PCT/CN2025/074597
(87) International publication number: WO 2025/162237

(57) **Abstract**

The present invention relates to a salt form and a crystal form of a 5-HT2A receptor inverse agonist, a preparation method therefor and the use thereof. Specifically, provided are a pharmaceutically acceptable salt of a compound of formula (I), a crystal form of a fumarate thereof, a preparation method therefor, and the use thereof for treating 5-HT2A receptor-related diseases.

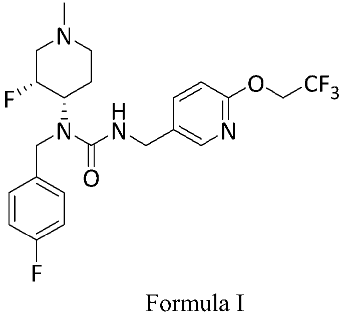

## Description

### TECHNICAL FIELD

The present disclosure belongs to the field of pharmaceutical chemistry and particularly relates to salt forms of a 5-HT_{2A} receptor inverse agonist, crystal forms, preparation methods therefor, and use thereof.

### BACKGROUND

Parkinson's disease is a common degenerative disease of the central nervous system, and previously, people often attach importance to the motor symptoms of PD, such as resting tremor, myotonia, bradykinesia, and postural gait disorder. Currently, there are about 7 million to 10 million patients with Parkinson's disease worldwide, and more than 50% of the patients have had mental symptoms. These mental symptoms are mainly manifested as hallucinations and delusions, which bring greater challenges to the treatment and care of patients with Parkinson's disease.

For a long time, antipsychotic drugs have been used primarily in clinical practice to treat hallucinations and delusions in patients with Parkinson's disease. First-generation antipsychotic drugs primarily inhibit dopamine D₂ receptors and are associated with severe extrapyramidal side effects. Second-generation antipsychotic drugs, in addition to inhibiting D₂ receptors, also more significantly inhibit specific 5-HT receptors, particularly the 5-HT_{2A} receptor, offering better safety, i.e., fewer extrapyramidal side effects compared to the first-generation antipsychotic drugs. However, since the second-generation antipsychotic drugs still have inhibitory activity against the D₂ receptors, they still cause extrapyramidal side effects. Additionally, such drugs also lead to the side effect of weight gain to varying degrees. In 2016, the U.S. FDA approved pimavanserin for marketing to treat hallucination and delusion symptoms associated with Parkinson's disease, making it the first drug approved for this indication.

Pimavanserin is a 5-HT_{2A} receptor inverse agonist that can eliminate the extrapyramidal and weight gain side effects associated with dopamine receptor inhibition caused by first- and second-generation antipsychotic drugs, offering better safety. 5-HT_{2A} is a major excitatory receptor subtype in the 5-HT receptor family, belonging to ligand-gated channels and G protein-coupled receptors. The function of the 5-HT_{2A} receptor is closely related to neuronal excitation, behavior effects, learning and memory, anxiety, and the like. This receptor is an important action target for antipsychotic drugs and the treatment of schizophrenia (Price, D.L., et al., Behavioural Pharmacology (2012), 23(4), 426-433). The 5-HT_{2A} receptor has intrinsic activity and is able to exert its effect even in the absence of an agonist. Pimavanserin, as a 5-HT_{2A} receptor inverse agonist, upon binding to the 5-HT_{2A} receptor, can inhibit the intrinsic activity of the receptor, making the receptor non-functional and resulting in an effect opposite to that of an agonist. It can still exhibit activity even in the absence of an agonist. In contrast, typical 5-HT_{2A} receptor antagonists cannot induce biological effects upon binding to the receptor and can only exhibit their activity by inhibiting agonists (WO2004064738A2). Thus, even if a compound has 5-HT_{2A} receptor antagonist activity, it may not necessarily have 5-HT_{2A} receptor inverse agonist activity.

PCT/CN2023/111052 provides a compound with a structure as shown below. The test results show that the compound has good 5-HT_{2A} receptor inverse agonist activity.

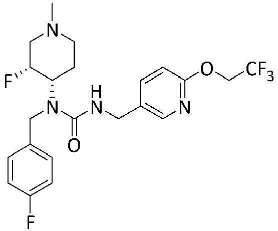

The pharmaceutical form of a compound, such as its crystal form or salt, often affects the chemical stability of the drug. Variations in crystallization and storage conditions may lead to changes in the crystal form structure of the compound and sometimes even result in the formation of other crystal forms. Generally, amorphous drug products lack a regular crystal form structure, which often leads to defects such as poor product stability, fine crystallization, filtration difficulties, proneness to agglomeration, and poor flowability. Given the importance of solid drug salt forms and crystal forms and their stability in clinical treatment, in-depth research on crystal forms of the compound of Formula I is of great significance for the development of drugs that are suitable for industrial production and have good biological activity.

### SUMMARY

In one aspect, the present disclosure provides a pharmaceutically acceptable salt of a compound of Formula I, wherein the pharmaceutically acceptable salt is selected from the group consisting of hydrochloride, sulfate, p-toluenesulfonate, maleate, phosphate, dihydroxybenzoate, mandelate, malate, succinate, acetate, hexanoate, palmitate, pamoate, citrate, L-tartrate, or fumarate, and is preferably fumarate,

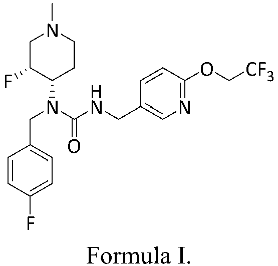

In an optional embodiment, the chemical ratio of the compound of Formula I to an acid molecule is about 1:2 to 2:1, and may be about 1:2, 1:1, or 2:1.

In an optional embodiment, the chemical ratio of the compound of Formula I to fumaric acid is about 1:1.

The present disclosure also provides a preparation method for the aforementioned pharmaceutically acceptable salt, which comprises the step of forming a salt by the compound of Formula I and an acid selected from the group consisting of hydrochloric acid, sulfuric acid, p-toluenesulfonic acid, maleic acid, phosphoric acid, dihydroxybenzoic acid, mandelic acid, malic acid, succinic acid, acetic acid, hexanoic acid, palmitic acid, pamoic acid, citric acid, L-tartaric acid, or fumaric acid. The solvent used for salt formation in the present disclosure is selected from the group consisting of one or more of water, methanol, ethanol, isopropanol, acetonitrile, tetrahydrofuran, ethyl acetate, and n-heptane. In one aspect, the present disclosure provides a Crystal Form A of the fumarate of the compound of Formula I, which has, in an X-ray powder diffraction pattern using Cu-Kα radiation, characteristic diffraction peaks at the following 2θ angles: 7.564±0.2°, 18.942±0.2°, 22.787±0.2°, and 26.629±0.2°. In some embodiments, the Crystal Form A of the fumarate of the compound of Formula I has, in an X-ray powder diffraction pattern using Cu-Kα radiation, characteristic diffraction peaks at the following 20 angles: 7.564±0.2°, 11.335±0.2°, 15.212±0.2°, 18.942±0.2°, 22.787±0.2°, 24.571±0.2°, and 26.629±0.2°.

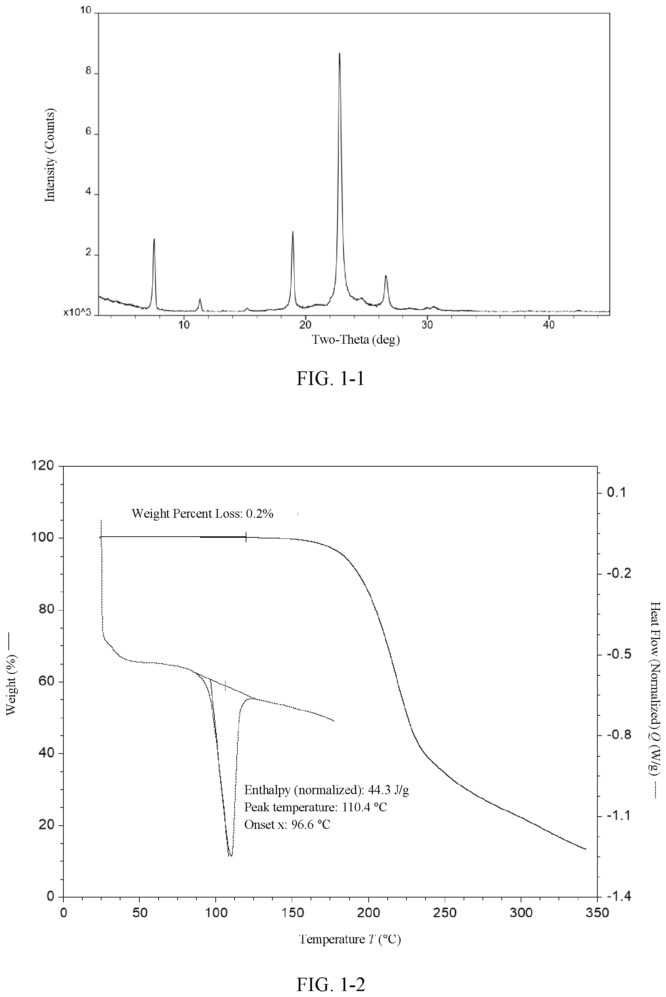

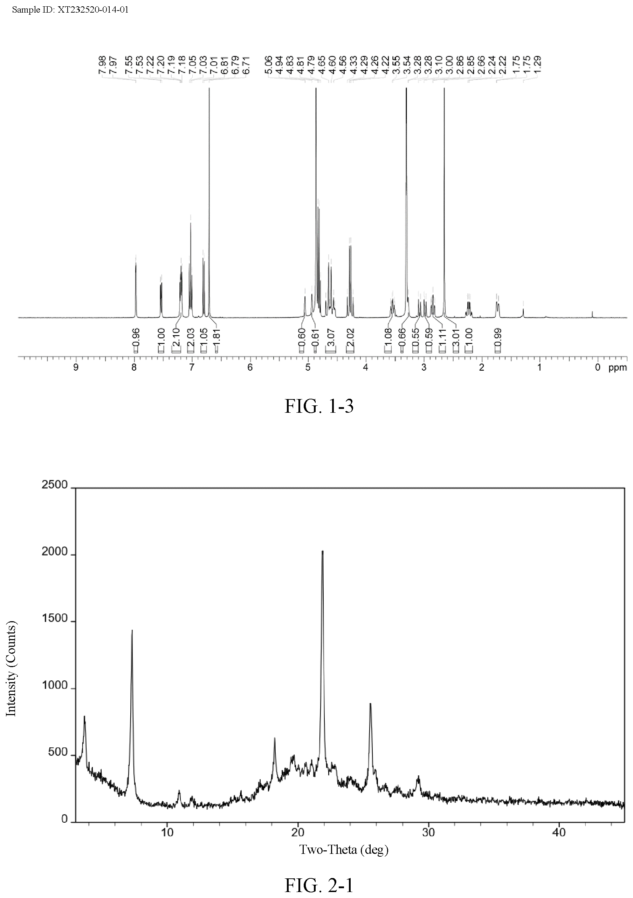

In some embodiments, the Crystal Form A of the fumarate of the compound of Formula I has an X-ray powder diffraction pattern using Cu-Kα radiation as shown in FIG. 1-1.

In some embodiments, the Crystal Form A of the fumarate of the compound of Formula I is an anhydrous crystal form.

In one aspect, the present disclosure provides a preparation method for the Crystal Form A of the fumarate of the compound of Formula I, which comprises the following step: subjecting the fumarate of the compound of Formula I to a method such as a suspension method or a cooling method to obtain the Crystal Form A.

In some embodiments, the present disclosure provides a preparation method for the Crystal Form A of the fumarate of the compound of Formula I, which comprises the following step: suspending the fumarate of the compound of Formula I in acetonitrile at room temperature to obtain the Crystal Form A.

In some embodiments, the present disclosure provides a preparation method for the Crystal Form A of the fumarate of the compound of Formula I, which comprises the following step: cooling the fumarate of the compound of Formula I in acetonitrile or a mixed solvent of toluene/acetonitrile to obtain the Crystal Form A.

In one aspect, the present disclosure provides a Crystal Form B of the fumarate of the compound of Formula I, which has, in an X-ray powder diffraction pattern using Cu-Kα radiation, characteristic diffraction peaks at the following 20 angles: 3.665±0.2°, 7.299±0.2°, 18.225±0.2°, 21.887±0.2°, and 25.564±0.2°.

In some embodiments, the present disclosure provides a Crystal Form B of the fumarate of the compound of Formula I, which has, in an X-ray powder diffraction pattern using Cu-Kα radiation, characteristic diffraction peaks at the following 20 angles: 3.665±0.2°, 7.299±0.2°, 18.225±0.2°, 21.887±0.2°, 25.564±0.2°, and 29.164±0.2°.

In some embodiments, the present disclosure provides a Crystal Form B of the fumarate of the compound of Formula I, which has, in an X-ray powder diffraction pattern using Cu-Kα radiation, characteristic diffraction peaks at the following 20 angles: 3.665±0.2°, 7.299±0.2°, 18.225±0.2°, 19.628±0.2°, 21.061±0.2°, 21.887±0.2°, 25.564±0.2°, 25.949±0.2°, and 29.164±0.2°.

In some embodiments, the present disclosure provides a Crystal Form B of the fumarate of the compound of Formula I, which has, in an X-ray powder diffraction pattern using Cu-Kα radiation, characteristic diffraction peaks at the following 20 angles: 3.665±0.2°, 7.299±0.2°, 10.921±0.2°, 11.923±0.2°, 18.225±0.2°, 19.628±0.2°, 21.061±0.2°, 21.887±0.2°, 25.564±0.2°, 25.949±0.2°, and 29.164±0.2°.

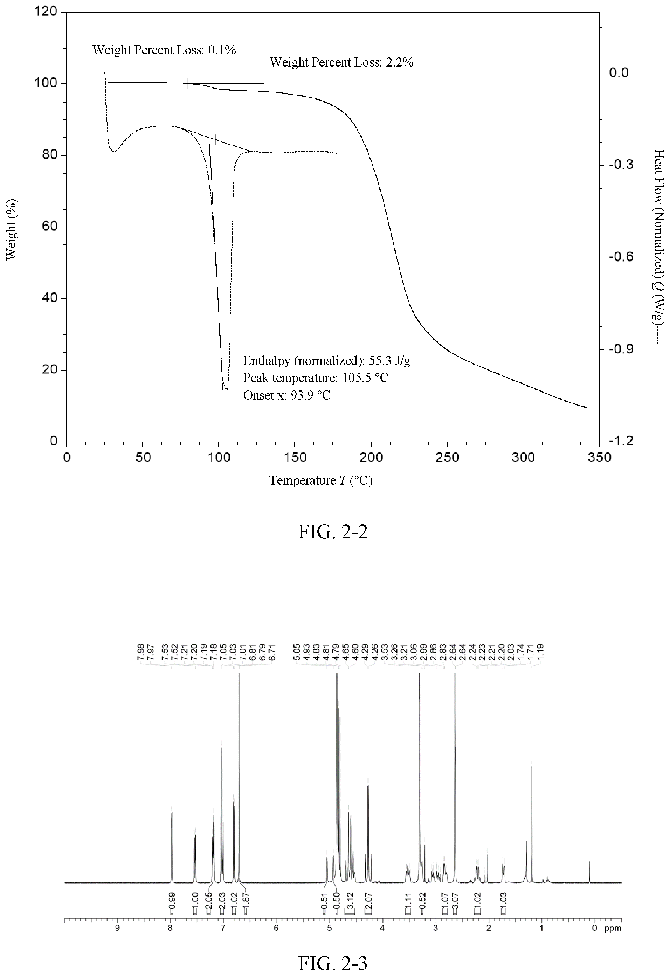

In some embodiments, the Crystal Form B of the fumarate of the compound of Formula I has an X-ray powder diffraction pattern using Cu-Kα radiation as shown in FIG. 2-1.

In some embodiments, the Crystal Form B of the fumarate of the compound of Formula I is an anhydrous crystal form.

In one aspect, the present disclosure provides a preparation method for the Crystal Form B of the fumarate of the compound of Formula I, which comprises the following step: subjecting the fumarate of the compound of Formula I to a method such as a suspension method or an anti-solvent crystallization method to obtain the Crystal Form B.

In some embodiments, the present disclosure provides a preparation method for the Crystal Form B of the fumarate of the compound of Formula I, which comprises the following steps: completely dissolving the fumarate of the compound of Formula I in a tetrahydrofuran solvent and then adding n-heptane dropwise until a solid is precipitated.

In some embodiments, the present disclosure provides a preparation method for the Crystal Form B of the fumarate of the compound of Formula I, which comprises the following step: suspending the fumarate of the compound of Formula I in a mixed solvent of acetone/n-heptane at room temperature to obtain the Crystal Form B.

In some embodiments, the present disclosure provides a preparation method for the Crystal Form B of the fumarate of the compound of Formula I, which comprises the following step: suspending the fumarate of the compound of Formula I in n-heptane at 50 °C to obtain the Crystal Form B.

In one aspect, the present disclosure provides a Crystal Form C of the fumarate of the compound of Formula I, which has, in an X-ray powder diffraction pattern using Cu-Kα radiation, characteristic diffraction peaks at the following 20 angles: 24.435±0.2°, 28.829±0.2°, and 38.312±0.2°.

In some embodiments, the Crystal Form C of the fumarate of the compound of Formula I has, in an X-ray powder diffraction pattern using Cu-Kα radiation, characteristic diffraction peaks at the following 20 angles: 21.245±0.2°, 24.435±0.2°, 28.829±0.2°, 38.312±0.2°, and 39.979±0.2°.

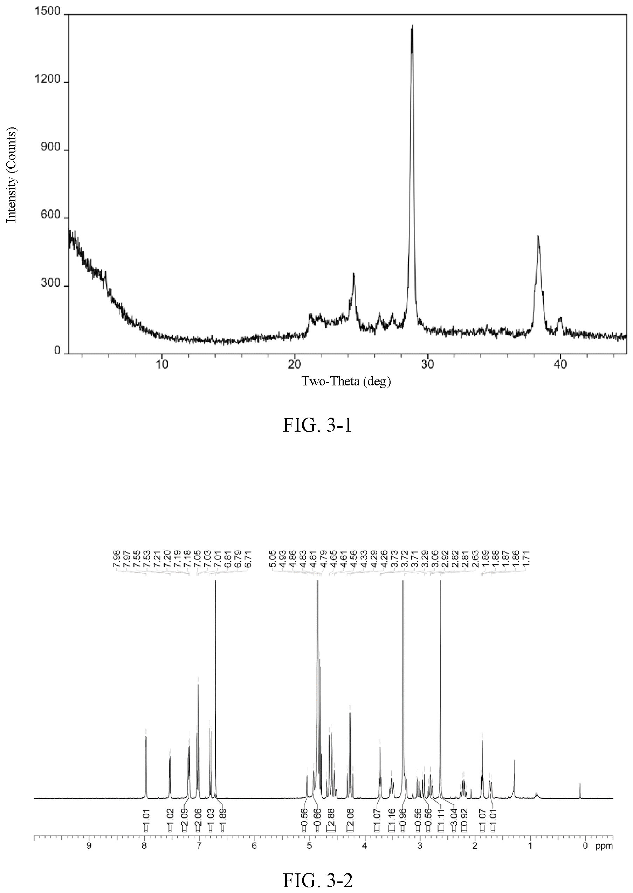

In some embodiments, the Crystal Form C of the fumarate of the compound of Formula I has an X-ray powder diffraction pattern using Cu-Kα radiation as shown in FIG. 3-1.

In one aspect, the present disclosure provides a preparation method for the Crystal Form C of the fumarate of the compound of Formula I, which comprises the following step: subjecting the fumarate of the compound of Formula I to a method such as a suspension method, an anti-solvent crystallization method, or a gas-phase diffusion method to obtain the Crystal Form C.

In some embodiments, the present disclosure provides a preparation method for the Crystal Form C of the fumarate of the compound of Formula I, which comprises the following steps: completely dissolving the fumarate of the compound of Formula I in a tetrahydrofuran solvent and then adding the solution dropwise to 10 volumes of dichloromethane.

In some embodiments, the present disclosure provides a preparation method for the Crystal Form C of the fumarate of the compound of Formula I, which comprises the following step: suspending the Crystal Form A or the Crystal Form B in toluene or dichloromethane at room temperature to obtain the Crystal Form C.

In some embodiments, the present disclosure provides a preparation method for the Crystal Form C of the fumarate of the compound of Formula I, which comprises the following step: suspending the fumarate of the compound of Formula I in toluene at 50 °C to obtain the Crystal Form C.

In one aspect, the present disclosure provides a Crystal Form D of the fumarate of the compound of Formula I, which has, in an X-ray powder diffraction pattern using Cu-Kα radiation, characteristic diffraction peaks at the following 2θ angles: 7.160±0.2°, 18.004±0.2°, 21.436±0.2°, and 25.082±0.2°. In some embodiments, the Crystal Form D of the fumarate of the compound of Formula I has, in an X-ray powder diffraction pattern using Cu-Kα radiation, characteristic diffraction peaks at the following 20 angles: 3.537±0.2°, 7.160±0.2°, 15.745±0.2°, 18.004±0.2°, 18.893±0.2°, 19.269±0.2°, 21.436±0.2°, and 25.082±0.2°.

In some embodiments, the Crystal Form D of the fumarate of the compound of Formula I has, in an X-ray powder diffraction pattern using Cu-Kα radiation, characteristic diffraction peaks at the following 2θ angles: 3.537±0.2°, 7.160±0.2°, 10.700±0.2°, 15.745±0.2°, 18.004±0.2°, 18.893±0.2°, 19.269±0.2°, 20.965±0.2°, 21.436±0.2°, 25.082±0.2°, and 28.762±0.2°.

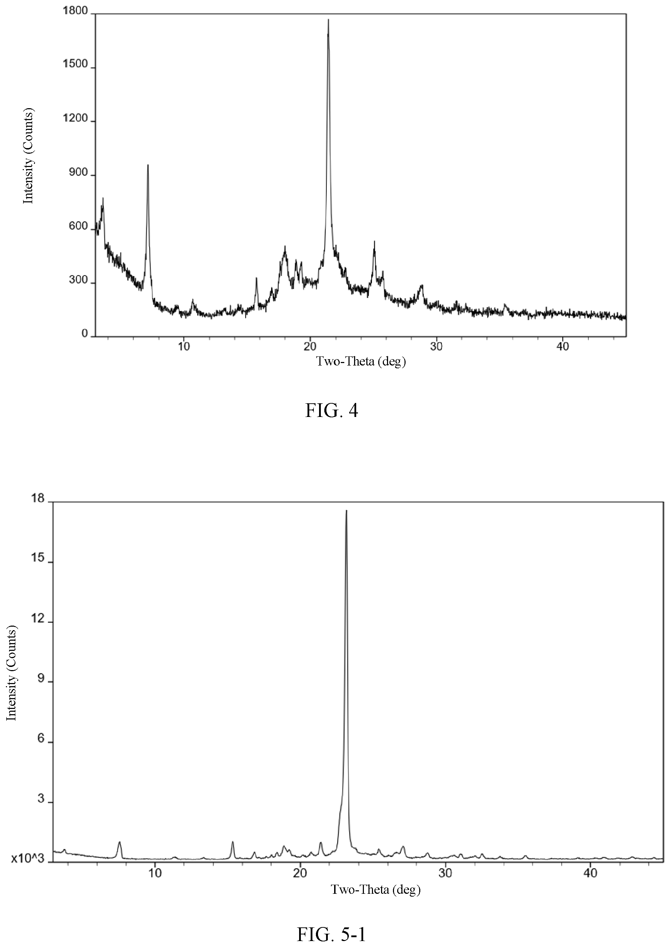

In some embodiments, the Crystal Form D of the fumarate of the compound of Formula I has an X-ray powder diffraction pattern using Cu-Kα radiation as shown in FIG. 4.

In one aspect, the present disclosure provides a preparation method for the Crystal Form D of the fumarate of the compound of Formula I, which comprises the following step: subjecting the fumarate of the compound of Formula I to a method such as a suspension method or an anti-solvent crystallization method to obtain the Crystal Form D.

In some embodiments, the present disclosure provides a preparation method for the Crystal Form D of the fumarate of the compound of Formula I, which comprises the following steps: completely dissolving the fumarate of the compound of Formula I in an ethyl acetate solvent and then adding the solution to n-heptane dropwise until a solid is precipitated.

In some embodiments, the present disclosure provides a preparation method for the Crystal Form D of the fumarate of the compound of Formula I, which comprises the following step: suspending the fumarate of the compound of Formula I in a solvent system of ethyl acetate/n-heptane or tetrahydrofuran/n-heptane at room temperature to obtain the Crystal Form D.

In some embodiments, the present disclosure provides a preparation method for the Crystal Form D of the fumarate of the compound of Formula I, which comprises the following step: suspending the Crystal Form B in a solvent system of tetrahydrofuran/n-heptane or ethyl acetate/n-heptane at room temperature to obtain the Crystal Form D.

In some embodiments, the present disclosure provides a preparation method for the Crystal Form D of the fumarate of the compound of Formula I, which comprises the following step: suspending the Crystal Form A in methyl tert-butyl ether at room temperature to obtain the Crystal Form D.

In one aspect, the present disclosure provides a Crystal Form E of the fumarate of the compound of Formula I, which has, in an X-ray powder diffraction pattern using Cu-Kα radiation, characteristic diffraction peaks at the following 20 angles: 7.570±0.2°, 15.357±0.2°, 18.889±0.2°, 21.436±0.2°, 23.168±0.2°, and 27.084±0.2°.

In some embodiments, the Crystal Form E of the fumarate of the compound of Formula I has, in an X-ray powder diffraction pattern using Cu-Kα radiation, characteristic diffraction peaks at the following 20 angles: 3.769±0.2°, 7.570±0.2°, 15.357±0.2°, 16.847±0.2°, 18.410±0.2°, 18.889±0.2°, 19.210±0.2°, 20.780±0.2°, 21.436±0.2°, 23.168±0.2°, 25.411±0.2°, 26.639±0.2°, 27.084±0.2°, 28.770±0.2°, 31.065±0.2°, and 32.507±0.2°.

In some embodiments, the Crystal Form E of the fumarate of the compound of Formula I has, in an X-ray powder diffraction pattern using Cu-Kα radiation, characteristic diffraction peaks at the following 2θ angles: 3.769±0.2°, 7.570±0.2°, 11.407±0.2°, 13.369±0.2°, 15.357±0.2°, 16.847±0.2°, 18.026±0.2°, 18.410±0.2°, 18.889±0.2°, 19.210±0.2°, 20.188±0.2°, 20.780±0.2°, 21.436±0.2°, 23.168±0.2°, 25.411±0.2°, 26.639±0.2°, 27.084±0.2°, 28.770±0.2°, 31.065±0.2°, 32.058±0.2°, 32.507±0.2°, 33.754±0.2°, 35.513±0.2°, 39.105±0.2°, 40.919±0.2°, 41.883±0.2°, 42.848±0.2°, and 44.342±0.2°.

In some embodiments, the Crystal Form E of the fumarate of the compound of Formula I has an X-ray powder diffraction pattern using Cu-Kα radiation as shown in FIG. 5-1.

In some embodiments, the Crystal Form E of the fumarate of the compound of Formula I is an anhydrous crystal form.

In some embodiments, the present disclosure provides a preparation method for the Crystal Form E of the fumarate of the compound of Formula I, which comprises the following step: drying the Crystal Form A under vacuum at 60-70 °C.

In one aspect, the present disclosure further provides a pharmaceutical composition, which comprises any one of the aforementioned pharmaceutically acceptable salts of the compound of Formula I or any one of the crystal forms of the fumarate of the compound of Formula I, and a pharmaceutically acceptable carrier. The pharmaceutical composition may be prepared in various pharmaceutically acceptable dosage forms, such as tablets, capsules, oral liquids, granules, injections, or various sustained-release and controlled-release preparations. The pharmaceutical composition may be administered orally or parenterally (e.g., intravenously, subcutaneously, or topically). The administration dose may be appropriately adjusted based on the patient's age, sex, and disease type, and the daily dose is generally about 1-200 mg.

In one aspect, the present disclosure further provides use of any one of the aforementioned pharmaceutically acceptable salts of the compound of Formula I or any one of the aforementioned crystal forms of fumarate of the compound of Formula I or the pharmaceutical composition in the preparation of a medicament for treating a 5-HT_{2A} receptor-related disease. The disease or symptom includes: schizophrenia, psychosis, schizoaffective disorder, manic disorder, psychotic depression, affective disorder, dementia, anxiety disorder, sleep disorder, appetite disorder, bipolar disorder, psychosis secondary to hypertension, migraine, hypertension, thrombosis, vasospasm, ischemia, motor tics, depression, major depressive disorder, anxiety, sleep disturbance and appetite disturbance, non-motor symptoms caused by Parkinson's disease (including delusion, hallucination, depression, anxiety, cognitive disorder, or sleep disorder), dementia-related mental diseases, negative symptoms of schizophrenia, Parkinson's disease, Huntington's chorea, Alzheimer's disease, spinocerebellar atrophy, Tourette's syndrome, Friedreich's ataxia, Machado-Joseph disease, Lewy body dementia, movement disorder, dystonia, myoclonus, tremor, progressive supranuclear palsy, and frontotemporal dementia; preferably Alzheimer's disease psychosis (ADP)-related hallucination and delusion, Parkinson's disease psychosis (PDP)-related hallucination and delusion, and negative symptoms of schizophrenia (NSS); or other disease states and conditions that are apparent to those skilled in the art.

### Definitions and Explanations

Unless otherwise specified, the following terms and phrases used herein are intended to have the following meanings. A particular term or phrase, when not specifically defined, should not be considered indefinite or unclear, but should be construed according to its common meaning. When referring to a trade name, it is intended to refer to its corresponding commercial product or its active ingredient.

The term "pharmaceutically acceptable" is described in the present disclosure in terms of compounds, compositions, and/or dosage forms that are, within the scope of sound medical judgment, suitable for contact with tissues of humans and animals without undue toxicity, irritation, allergic response, or other problems or complications, and commensurate with a reasonable benefit/risk ratio.

The compounds of the present disclosure may have asymmetric carbon atoms (optical centers) or double bonds. Racemates, diastereoisomers, geometric isomers, and individual isomers all fall within the scope of the present disclosure.

The compounds of the present disclosure may be present in particular geometric or stereoisomeric forms. All such compounds are contemplated in the present disclosure, including cis and trans isomers, (-)- and (+)-enantiomers, (*R*)- and (*S*)-enantiomers, diastereoisomers, (*D*)-isomers, (*L*)-isomers, and racemic mixtures and other mixtures thereof, such as enantiomerically or diastereoisomerically enriched mixtures, all of which fall within the scope of the present disclosure. Additional asymmetric carbon atoms may be present in substituents such as alkyl. All such isomers and mixtures thereof fall within the scope of the present disclosure.

The term "pharmaceutically acceptable carrier" refers to any preparation or carrier medium that is capable of delivering an effective amount of the active substance of the present disclosure, does not interfere with the biological activity of the active substance, and has no toxic or side effects on a host or patient; representative carriers include, but are not limited to: binders, fillers, lubricants, disintegrants, wetting agents, dispersing agents, solubilizers, suspending agents, etc.

The present disclosure is intended to include all isotopes of atoms present in the compounds of the present disclosure. Isotopes include those atoms having the same atomic number but different mass numbers. As a general and non-limiting example, isotopes of hydrogen include deuterium and tritium. Isotopes of carbon include ¹³C and ¹⁴C. Isotopically labeled compounds of the present disclosure may generally be prepared by conventional techniques known to those skilled in the art or by methods similar to those described herein using an appropriate isotopically labeled reagent in place of the non-labeled reagent otherwise used.

### BRIEF DESCRIPTION OF THE DRAWINGS

FIGs. 1-1, 1-2, and 1-3 show an XRPD pattern, TGA/DSC thermograms, and a ¹H NMR spectrum of the Crystal Form A of the fumarate of the compound of Formula I, respectively.
FIGs. 2-1, 2-2, and 2-3 show an XRPD pattern, TGA/DSC thermograms, and a ¹H NMR spectrum of the Crystal Form B of the fumarate of the compound of Formula I, respectively.
FIGs. 3-1 and 3-2 show an XRPD pattern and a ¹H NMR spectrum of the Crystal Form C of the fumarate of the compound of Formula I, respectively.
FIG. 4 shows an XRPD pattern of the Crystal Form D of the fumarate of the compound of Formula I.
FIGs. 5-1, 5-2, and 5-3 show an XRPD pattern, TGA/DSC thermograms, and a ¹H NMR spectrum of the Crystal Form E of the fumarate of the compound of Formula I, respectively.
FIG. 6 shows the number of head twitches in SD rats 1.0-1.5 h after single intragastric administration (where # indicates P < 0.05, as compared to the Control group; * indicates P < 0.05 and ** indicates P < 0.01, as compared to the Model group).

### DETAILED DESCRIPTION

The present disclosure is further illustrated below with reference to specific examples and test examples, but these examples do not limit the scope of the present disclosure in any way.

Test conditions for the instruments used in the experiments:

### Nuclear magnetic resonance analysis (¹H NMR)

Solid samples were dissolved in MeOH-d4 solvent and analyzed using a Bruker AVANCE NEO 400 nuclear magnetic resonance spectrometer (Bruker, GER).

### X-ray powder diffraction (XRPD)

The solid samples were analyzed using an X-ray powder diffractometer Bruker D8 Advance (Bruker, GER). The 20 scanning angle was from 3° to 45°, the scanning step size was 0.02°, and the exposure time was 0.08 s. The test method was Cu target Kα1 radiation at a voltage of 40 kV and a current of 40 mA, and a zero-background sample pan was used.

### Thermogravimetric analysis (TGA)

The models of the thermogravimetric analyzers were TA Discovery 55 (TA, US) and TA Discovery 550 (TA, US). A 2-5 mg sample was placed in an equilibrated open aluminum sample pan and automatically weighed in a TGA furnace. The sample was heated to the final temperature at a rate of 10 °C/min with a nitrogen purging speed of 60 mL/min at the sample and a nitrogen purging speed of 40 mL/min at the balance.

### Differential Scanning Calorimetry (DSC)

The model of the differential scanning calorimeter was TA Discovery 250 (TA, US). A 1-2 mg sample was accurately weighed out, then placed in a perforated DSC Tzero sample pan, and heated to the final temperature at a rate of 10 °C/min with a nitrogen purging speed of 50 mL/min in the furnace.

### Example 1. Synthesis of Compound of Formula I

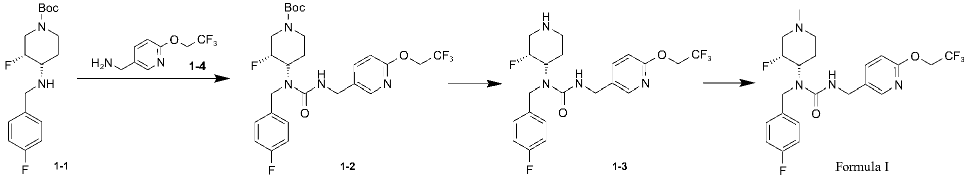

### 1.1: synthesis of compound 1-2

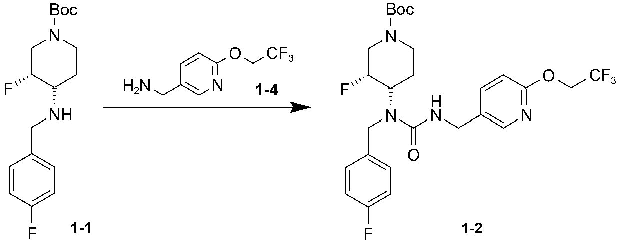

Tetrahydrofuran (15 mL) and compound **1-4** (1.04 g, 1.00 *eq,* HCl) were sequentially added to a 100 mL three-necked flask, and diisopropylethylamine (1.11 g, 2.00 *eq)* was added dropwise in a nitrogen atmosphere. The mixture was stirred at 20 °C for 15 min. The reaction solution was then cooled to 0 °C, and N,N'-carbonyldiimidazole (765 mg, 1.10 *eq)* was added thereto in portions. The reaction was monitored to find that compound **1-4** had completely reacted. Compound **1-1** (1.40 g, *1.00 eq)* was dissolved in tetrahydrofuran (5 mL), and the resulting solution was slowly added dropwise to the reaction solution. The mixture was maintained at 25 °C and stirred for 12 h. Water (100 mL) was added to the reaction solution, and the mixture was extracted three times with ethyl acetate (50 mL each time). The organic phases were combined, washed with saturated brine (80 mL), dried, and concentrated to dryness to obtain a crude product. The crude product was purified by column chromatography to obtain compound **1-2** (colorless oily substance, 966 mg, yield: 38.8%).

### 1.2: synthesis of compound 1-3

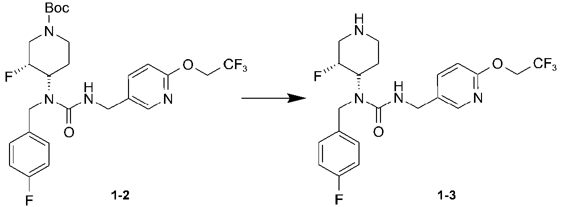

Anhydrous dichloromethane (27 mL), compound **1-2** (966 mg, 1.00 *eq),* and trifluoroacetic acid (15.4 g, 78.0 *eq)* were sequentially added to a 50 mL single-necked flask, and the mixture was maintained at 25 °C and stirred for 20 min. The reaction solution was concentrated to dryness by rotary evaporation to obtain a crude product of compound **1-3** (light yellow oily substance, 850 mg, yield: 91.4%).

### 1.3: synthesis of compound of Formula I

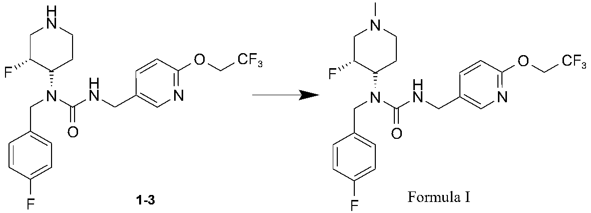

Tetrahydrofuran (8 mL), compound **1-3** (800 mg, 1.00 *eq),* sodium cyanoborohydride (219 mg, 2.00 *eq),* and formaldehyde (212 mg, 37%, 1.50 *eq)* were sequentially added to a 50 mL single-necked flask. The mixture was maintained at 25 °C and stirred for 12 h. Water (50 mL) was added to the reaction solution, and the mixture was extracted three times with ethyl acetate (30 mL each time). The organic phases were combined, washed with saturated brine (40 mL), dried, and concentrated to dryness, and the residue was purified by column chromatography (petroleum ether/ethyl acetate = 1/0 to 5/1) to obtain the **compound of Formula** I (white solid, 643 mg, yield: 78.1%). ¹H NMR (400 MHz, CDCl₃) *δ* 7.87 (d, *J =* 1.9 Hz, 1H), 7.35 (dd, *J =* 2.3, 8.5 Hz, 1H), 7.17 - 7.08 (m, 2H), 7.00 (br t, *J* = 8.5 Hz, 2H), 6.75 (d, *J =* 8.5 Hz, 1H), 5.17 - 4.99 (m, 1H), 4.94 (br t, *J =* 5.1 Hz, 1H), 4.71 (d, *J* = 8.6 Hz, 2H), 4.56 - 4.43 (m, 2H), 4.30 - 4.17 (m, 2H), 3.81 - 3.57 (m, 2H), 3.22 - 2.91 (m, 2H), 2.80 (s, 3H), 2.60 - 2.45 (m, 1H), 1.79 (br d, *J* = 11.8 Hz, 1H). MS(ESI) m/z: 473.2[M+1].

### Example 2. Preparation of Fumarate of Compound of Formula I

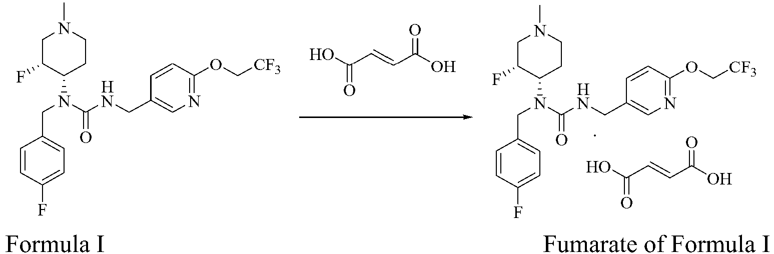

Acetonitrile (513 mL), the compound of Formula I (49.3 g, 1.00 *eq),* and fumaric acid (13.46 g, 1.00 *eq)* were sequentially added to a 1 L single-necked flask. The mixture was maintained at 55±5 °C and stirred for 2-3 h. The mixture was then cooled to 20±5 °C, maintained at this temperature, and subjected to crystallization for 2-3 h. The mixture was then filtered, dried in a vacuum drying oven at 30 °C for 1 h, and heated to 60 °C for 6 h to obtain the **fumarate of the compound of Formula I** (white solid, 55.1 g, yield: 89.7%). ¹H NMR (600 MHz, MeOD) δ 7.88 (1H, t, *J*=1.8, 2.4 Hz), 7.44 (1H, dd, *J*=2.4, 8.4 Hz), 7.10 (2H, dd, *J*=5.4, 8.4 Hz), 6.92 (2H, dd, *J*=6.6, 8.4 Hz), 6.70 (1H, dd, *J*=0.6, 8.4 Hz), 6.59 (2H, s), 4.92 (1H, m), 4.72 (2H, m), 4.50 (1H, m), 4.48 (2H, m), 4.17 (2H, m), 3.52 (1H, m), 3.27 (1H, m), 3.05 (1H, m), 2.85 (1H, m), 2.62 (3H, s), 2.15 (1H, m), 1.65 (1H, m).

### Example 3. Preparation of Crystal Form A of Fumarate of Compound of Formula I

The fumarate of the compound of Formula I (1.5 g) was weighed out, and acetonitrile (37 mL) was added thereto. The mixture was stirred at 50 °C until the sample was completely dissolved. After the clear solution was suspended overnight at room temperature, a large amount of solid was precipitated. The suspension was centrifuged and the resulting solid was dried under vacuum at room temperature overnight. The resulting solid was dried under vacuum at 40 °C overnight to obtain the Crystal Form A of the fumarate of the compound of Formula I (700 mg).

The product was characterized by XRPD, ¹H NMR, TGA, and DSC. The XRPD pattern is as shown in FIG. 1-1, and the characteristic peak positions are as shown in Table 1. The TGA/DSC results (FIG. 1-2) show that there was a weight loss of 0.2% during heating to 120 °C, decomposition might occur at ≥ 180 °C, and an endothermic signal was detected at 110.4 °C (peak temperature). The ¹H NMR results (FIG. 1-3) show that there was no significant solvent signal peak, a fumaric acid peak was detected at 6.71 ppm, and the salt-forming ratio was 1:0.9. In conclusion, the Crystal Form A is an anhydrate crystal form.

**Table 1. XRPD pattern analysis data for the Crystal Form A of the fumarate of the compound of Formula I**

| No. | 2θ±0.2 (°) | Relative intensity (%) | No. | 2θ±0.2 (°) | Relative intensity (%) |
|---|---|---|---|---|---|
| 1 | 7.564 | 27.6 | 5 | 22.787 | 100 |
| 2 | 11.335 | 4.8 | 6 | 24.571 | 2.8 |
| 3 | 15.212 | 1.2 | 7 | 26.629 | 12.2 |
| 4 | 18.942 | 30.5 | - | - | - |

### Example 4. Preparation of Crystal Form B of Fumarate of Compound of Formula I

The fumarate of the compound of Formula I (20 mg) was weighed out, and tetrahydrofuran (0.1 mL) was added dropwise at room temperature to completely dissolve the sample. Then, n-heptane (0.2 mL) was added dropwise until a solid was precipitated. The mixture was stirred at room temperature for about 30 min, and then the system from which a solid had precipitated was centrifuged. The solid was dried under vacuum at room temperature to obtain the Crystal Form B of the fumarate of the compound of Formula I.

The crystal form B was characterized by XRPD, ¹H NMR, TGA, and DSC. The XRPD pattern is as shown in FIG. 2-1, and the characteristic peak positions are as shown in Table 2. The TGA/DSC results (FIG. 2-2) show that the Crystal Form B had a weight loss of 0.1% during heating to 80 °C and a weight loss of 2.2% during heating from 80 °C to 130 °C, might decompose at ≥ 180 °C; and had an endothermic signal at about 105.5 °C (peak temperature). The ¹H NMR spectrum is as shown in FIG. 2-3. There was no significant solvent residue in the sample, a fumaric acid peak was detected at 6.71 ppm, and the salt-forming ratio is 1:0.9. In conclusion, the Crystal Form B is an anhydrous crystal form.

**Table 2. XRPD pattern analysis data for the Crystal Form B of the fumarate of the compound of Formula I**

| No. | 2θ±0.2 (°) | Relative intensity (%) | No. | 2θ±0.2 (°) | Relative intensity (%) |
|---|---|---|---|---|---|
| 1 | 3.665 | 22.2 | 7 | 21.061 | 10.0 |
| 2 | 7.299 | 72.4 | 8 | 21.887 | 100.0 |
| 3 | 10.921 | 6.6 | 9 | 25.564 | 37.8 |
| 4 | 11.923 | 4.5 | 10 | 25.949 | 9.7 |
| 5 | 18.225 | 22.9 | 11 | 29.164 | 7.8 |
| 6 | 19.628 | 12.3 | - | - | - |

### Example 5. Preparation of Crystal Form C of Fumarate of Compound of Formula I

The fumarate of the compound of Formula I (20.1 mg) was weighed out, and tetrahydrofuran (0.1 mL) was added dropwise at room temperature to completely dissolve the sample. The clear solution was added dropwise to dichloromethane (1.0 mL). The mixture was stirred at room temperature for about 30 min and centrifuged, and the resulting solid was dried under vacuum at room temperature overnight to obtain the Crystal Form C of fumarate of the compound of Formula I. The Crystal Form C was characterized by XRPD and ¹H NMR. The XRPD pattern is as shown in FIG. 3-1, and the characteristic peak positions are as shown in Table 3. The ¹H NMR spectrum is as shown in FIG. 3-2. Signal peaks of tetrahydrofuran were detected at 3.74-3.71 ppm and 1.88-1.85 ppm. According to the integration results, the ratio of the compound to tetrahydrofuran was 1:0.3, and the crude estimated mass proportion was about 3.5%. A fumaric acid peak was detected at 6.71 ppm, and the salt-forming ratio was 1:0.95.

**Table 3. XRPD pattern analysis data for the Crystal Form C of the fumarate of the compound of Formula I**

| No. | 2θ±0.2 (°) | Relative intensity (%) | No. | 2θ±0.2 (°) | Relative intensity (%) |
|---|---|---|---|---|---|
| 1 | 21.245 | 6.6 | 4 | 38.312 | 33.9 |
| 2 | 24.435 | 18.4 | 5 | 39.979 | 5.7 |
| 3 | 28.829 | 100.0 | - | - | - |

### Example 6. Preparation of Crystal Form D of Fumarate of Compound of Formula I

The fumarate of the compound of Formula I (20.8 mg) was weighed out and suspended in an ethyl acetate/n-heptane (v/v, 1:4) system (2.0 mL) at room temperature for 7 days to obtain the Crystal Form D of the fumarate of the compound of Formula I. The Crystal Form D was characterized by XRPD. The XRPD pattern is as shown in FIG. 4, and the characteristic peak positions are as shown in Table 4.

**Table 4. XRPD pattern analysis data for the Crystal Form D of the fumarate of the compound of Formula I**

| No. | 2θ±0.2 (°) | Relative intensity (%) | No. | 2θ±0.2 (°) | Relative intensity (%) |
|---|---|---|---|---|---|
| 1 | 3.537 | 11.9 | 7 | 19.269 | 10.5 |
| 2 | 7.160 | 47.6 | 8 | 20.965 | 9.9 |
| 3 | 10.700 | 5.7 | 9 | 21.436 | 100.0 |
| 4 | 15.745 | 11.8 | 10 | 25.082 | 19.7 |
| 5 | 18.004 | 19.4 | 11 | 28.762 | 7.9 |
| 6 | 18.893 | 11.8 | - | - | - |

### Example 7. Preparation of Crystal Form E of Fumarate of Compound of Formula I

Crystal Form A of the fumarate of the compound of Formula I (20.1 mg) was weighed out and dried under vacuum at 60 °C overnight to obtain the Crystal Form E of the fumarate of the compound of Formula I (yield: 100.0%; purity: 99.96%).

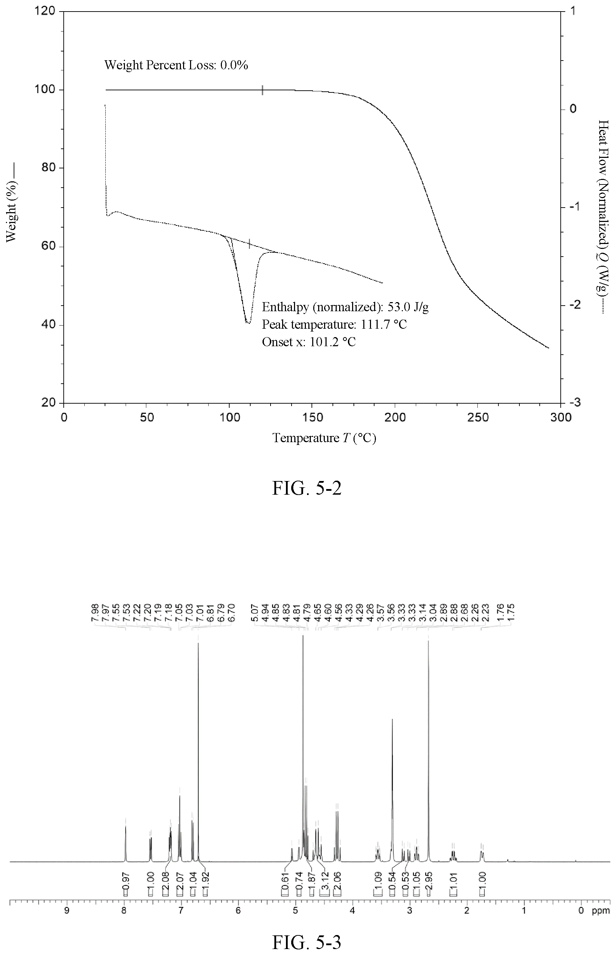

The Crystal Form E was characterized by XRPD, DSC, TGA, and ¹H NMR. The XRPD pattern is as shown in FIG. 5-1, and the characteristic peak positions are as shown in Table 5. The TGA/DSC results (FIG. 5-2) show that there was no weight loss during heating to 120 °C, decomposition might occur at 190 °C or higher, and an endothermic signal was detected at about 111.7 °C (peak temperature). The ¹H NMR results (FIG. 5-3) show that there was no significant solvent signal peak, a fumaric acid peak was detected at 6.70 ppm, and the salt-forming ratio was 1:0.96. In conclusion, the Crystal Form E is an anhydrate crystal form.

**Table 5. XRPD pattern analysis data for the Crystal Form E of the fumarate of the compound of Formula I**

| No. | 2θ±0.2 (°) | Relative intensity (%) | No. | 2θ±0.2 (°) | Relative intensity (%) |
|---|---|---|---|---|---|
| 1 | 3.769 | 1.1 | 15 | 25.411 | 2.0 |
| 2 | 7.570 | 4.7 | 16 | 26.639 | 1.2 |
| 3 | 11.407 | 0.8 | 17 | 27.084 | 3.3 |
| 4 | 13.369 | 0.6 | 18 | 28.770 | 1.5 |
| 5 | 15.357 | 5.2 | 19 | 31.065 | 1.0 |
| 6 | 16.847 | 1.9 | 20 | 32.058 | 0.5 |
| 7 | 18.026 | 0.9 | 21 | 32.507 | 1.3 |
| 8 | 18.410 | 1.5 | 22 | 33.754 | 0.7 |
| 9 | 18.889 | 3.4 | 23 | 35.513 | 0.9 |
| 10 | 19.210 | 2.0 | 24 | 39.105 | 0.4 |
| 11 | 20.188 | 0.4 | 25 | 40.919 | 0.5 |
| 12 | 20.780 | 1.3 | 26 | 41.883 | 0.3 |
| 13 | 21.436 | 3.9 | 27 | 42.848 | 0.7 |
| 14 | 23.168 | 100.0 | 28 | 44.342 | 0.4 |

### Test Example 1. Assay for 5-HT2A Receptor Inverse Agonist Activity

### 1.1 Experimental materials:

Cell line: adherent cells NIH3T3-5-HT_{2A} R
Cell culture medium: DMEM + 10% FBS (purchased from GBICO)
Cell culture plate: 96-well white/clear bottom plate (purchased from Perkin Elmer)
Assay kit: Bright-Glo^{™} Luciferase (purchased from Promega)
Assay instrument: BioTek multimode microplate reader

### 1.2 Test drugs

Pimavanserin: purchased from MCE (MedChemExpress)
Compound of Formula I: prepared according to the example described above

### 1.3 Experimental method:

NIH₃T3-5HT_{2A} R cells in the logarithmic growth phase were seeded into a 96-well white/clear bottom plate at a density of 1000 cells/well and then cultured overnight in an incubator at 37 °C with 5% CO₂. The next day, the test compound was diluted with PBS at a 3.16-fold concentration gradient to obtain 9 concentrations (the highest concentration of the test compound was 10 µM) and added to the cells. Duplicate wells were set up for each concentration. PBS was used as a negative control group, and pimavanserin at the same concentration was used as a positive control group. After the drug addition, the cells were cultured in the incubator at 37 °C with 5% CO₂ for 120 h. On the sixth day, a Bright-Glo^{™} Luciferase reagent was added to the cells in a volume equal to that of the cell culture medium. The plate was then incubated in the dark at room temperature for 20 min, with the plate shaker vibrating once every 5 min. The luminescence intensity was detected using a microplate reader, and the cell inhibition rate was calculated. Data were processed using GraphPad Prism 7.0 to obtain the cell inhibition rate curve and calculate IC₅₀. The test results are shown in Table 6. 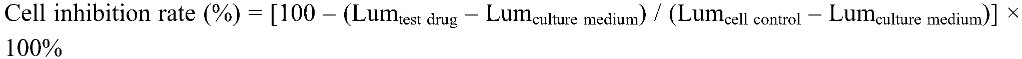 Cell inhibition rate (%) = [100 - (Lumtest drug - Lumculture medium) / (Lumcell control - Lumculture medium)] × 100%

### Test Example 2. hERG Inhibitory Activity

1. Test materials and instruments
1.1 Positive control compound
   Name: cisapride
1.2 Vehicle
   Name: DMSO (dimethyl sulfoxide)
1.3 Cells
   Species & strain: CHO-hERG cell line (Chinese hamster ovary cells stably expressing hERG channels)
   Culture medium: 90% F12, 10% fetal bovine serum, 100 µg/mL G418, and 100 µg/mL Hygromycin B
   Culture conditions: incubator at 37 °C with 5% CO₂
   Cryopreservation condition: liquid nitrogen
1.4 Experimental instruments
   Patch clamp amplifier (Axoclamp 200B, Multiclamp 700B, Axon, USA)
   Digital-to-analog converter (DigiData 1440A, DigiData 1550B, Axon, USA)
   Inverted microscope (IX51, IX71, Olympus, Japan)
   Rapid administration system (RSC-200, Bio-Logic, France)
   Micromanipulator (MX7600R, Syskiyou, USA)
   Electrode puller (P-97, Sutter, USA)
   Glass electrode (BF150-86-10, Sutter, USA)
   Vibration isolation table and shielding mesh (63-534, TMC, USA)
   Data acquisition and analysis software (pClamp 10, Axon, USA)
   Carbon dioxide incubator (HERAcell 150i, Thermo, USA)
   Biosafety cabinet (MODEL 1384, Thermo, USA)
   Water purification system (Milli Q, Millipore, USA)

2. Experimental method
2.1 Cell culture and treatment
CHO cells stably expressing hERG were cultured in a cell culture dish with a diameter of 35 mm and incubated in an incubator at 37 °C with 5% CO₂. The cells were passaged at a ratio of 1:5 every 48 h. On the day of the test, the cell culture medium was pipetted off, and the cells were rinsed once with the extracellular fluid. Then, a 0.25% Trypsin-EDTA (Invitrogen) solution was added, and the cells were digested at room temperature for 3-5 min. The digestion solution was then pipetted off, and the cells were resuspended in the extracellular fluid and transferred to an experimental dish for electrophysiological recording for later use.
2.2 Compound preparation
On the day of the test, the compound was diluted with DMSO to an intermediate concentration. Then, 10 µL of the intermediate-concentration compound was transferred to 4990 µL of extracellular fluid, achieving a 500-fold dilution to obtain the final concentration to be tested.
Preparation of positive control compound cisapride: 10 µL of 150 µM cisapride DMSO mother solution was transferred to 4990 µL of extracellular fluid, achieving a 500-fold dilution to obtain the final concentration (300 nM) to be tested.

2.3 Electrophysiological recording process
hERG potassium channel currents were recorded at room temperature by the whole-cell patch clamp technique in the CHO (Chinese hamster ovary) cells stably expressing hERG potassium channels. Glass microelectrodes were formed by pulling a glass electrode blank (BF150-86-10, Sutter) using a puller. The tip resistance after perfusion of electrode internal solution was about 2-5 MΩ. The glass microelectrodes could be connected to a patch clamp amplifier after being inserted into amplifier probes. The clamping voltage and data recording were controlled and recorded by a computer with a pClamp 10 software, with a sampling frequency of 10 kHz and a filtering frequency of 2 kHz. After achieving the whole-cell recording, the cells were clamped at -80 mV. To evoke the hERG potassium current (I_{hERG}), a step voltage was applied to depolarize from -80 mV to +20 mV for 2 s, followed by repolarization to -50 mV for 1 s and then returning to -80 mV. This voltage stimulation was applied every 10 s, and after it was determined that the hERG potassium currents were stable (1 min), the administration process was started. Each test concentration of the compound was applied for at least 1 min, and at least 2 cells (n ≥ 2) were tested for each concentration.
2.4 Data processing and analysis
Data analysis was performed using the pClamp 10 software and GraphPad Prism 5.0 software.
The degree of inhibition of hERG potassium current (the peak hERG tail current evoked at -50 mV) by different compound concentrations was calculated using the following formula: $Inhibition % = \left[1-\left(I/Io\right)\right] \times 100 %$ where Inhibition % represents the percentage of inhibition of hERG potassium current by the compound, and I and Io represent the amplitudes of hERG potassium current after administration and before administration, respectively.
The IC₅₀ of the compound was calculated using the equation below, based on the fitting performed by GraphPad Prism 5 software. The test results are shown in Table 6. $Y = Bottom + \left(Top-Bottom\right) / \left(1+{10}^{∧}\left(\left({LogIC}_{50}-X\right)\times HillSlope\right)\right)$ where X is the Log value of the test concentration of the test sample, Y is the inhibition percentage at the corresponding concentration, and Bottom and Top are the minimum and maximum inhibition percentages, respectively.

**Table 6. In vitro test results for the compound of Formula I**

| Compound No. | 5-HT_{2A} inverse agonist activity IC₅₀ (nM) | hERG inhibitory activity IC₅₀ (µM) |
|---|---|---|
| Pimavanserin | 344 | 1.14 |
| Formula I | 76.4 | 5.19 |

The results show that: the compound of Formula I exhibited better 5-HT_{2A} inverse agonist activity compared to pimavanserin, and had lower cardiotoxicity.

### Test Example 3. Evaluation of In Vitro Liver Microsomal Stability

1. Solution preparation
   1) Preparation of test sample working solution: A test sample was diluted to 100 µM with methanol.
   2) Preparation of liver microsome working solution: Liver microsomes were diluted to 0.56 mg/mL with 100 mM phosphate-buffered saline.
   3) Preparation of working solution of reduced nicotinamide adenine dinucleotide phosphate (NADPH): A proper amount of NADPH was weighed out and diluted to 20 mM with phosphate-buffered saline, and then an equal volume of a 60 mM MgCl₂ solution was added.
   4) Preparation of stop solution: Tolbutamide was diluted to 20 ng/mL with acetonitrile to serve as a stop solution containing an internal standard.
2. Incubation process
   1) Anti-adsorption EP tubes for incubation were prepared and labeled with species, test samples, control samples (testosterone and dextromethorphan), time points (0 min, 5 min, 10 min, 20 min, 30 min, 60 min, Blank60, and NCF60), etc.
   2) 2 µL of test or control sample working solution and 178 µL of liver microsome working solution were added to each tube. For the Blank tube, 2 µL of acetonitrile was added instead of the test sample. The tubes were pre-incubated in a water bath at 37 °C for about 10 min, with 3 replicates set up for each sample.
   3) After the pre-incubation was completed, 20 µL of NADPH working solution was added to each tube (except for the 0 min and NCF60 tubes) to start the reaction. For the NCF60 tube, 20 µL of phosphate-buffered saline (containing 30 mM MgCl₂) was added. In the incubation system, the final concentration of the test sample or control sample was 1 µM, the final concentration of liver microsomes was 0.5 mg/mL, the final concentration of NADPH was 1 mM, and the final concentration of MgCl₂ was 3 mM.
   4) For the 0 min sample, 600 µL of the stop solution was added, and the NADPH working solution was then added. For the other samples, after corresponding periods of incubation, 600 µL of the stop solution was added to stop the reaction.
   5) After the reaction was stopped, each sample was vortexed for 30 s and then centrifuged at 13500 rpm for 10 min. Then, 100 µL of the supernatant was transferred to an EP tube, and 100 µL of Milli-Q water was added. The mixture was well mixed by vortexing and analyzed by LC-MS/MS.
   6) Testosterone and dextromethorphan were tested as positive control samples under the same conditions to assess the stability and reliability of the system.
3. Data analysis

The remaining percentage of the test sample after 60 min was tested, and the test results are shown in Table 7.

**Table 7. Test data for the compound of Formula I in human and rat liver microsomes**

| Compound | Species | Remaining amount at 60 min (%) |
|---|---|---|
| Pimavanserin | Dog | 68.5 |
| | Human | 70.7 |
| Formula I | Dog | 81.75 |
| | Human | 42.89 |

The results show that the compound of Formula I had better stability in dog liver microsome than pimavanserin *in vitro* and exhibited better potential to be manufactured as a drug.

### Test Example 4. Evaluation of In Vivo Pharmacodynamics

### 1. Experimental protocol

Based on the *in vivo* pharmacokinetic data for the series of compounds, the drug reached Cₘₐₓ 1.0-1.5 h after single intragastric administration in SD rats. At this time point, 4-iodo-2,5-dimethoxy-A-methyl-phenethylamine hydrochloride (i.e., DOI, a 5-HT_{2A} receptor agonist) was intraperitoneally injected into the SD rats at a dose of 2.5 mg/kg to induce head twitch behavior in the SD rats, thereby establishing a model.

The low, medium, and high dose groups for the compound of Formula I were set to 0.22 mg/kg, 0.66 mg/kg, and 2.0 mg/kg, respectively. The dose of pimavanserin tartrate (Pim-T) was set at 0.7 mg/kg. The routes of administration of the compound of Formula I and Pim-T were both oral intragastric administration.

### 2. Experimental grouping and administration

The compounds were dissolved in DMSO:20% solutol (5%:95%). SD rats (200-250 g) were randomly divided into the following 6 groups according to the body weight: a vehicle control (Control) group, a model (Model) group, a Pim-T 0.7 mg/kg group, and the compound of Formula I 0.22 mg/kg, 0.66 mg/kg, and 2.0 mg/kg groups, with 7 animals in each group. Information on animal grouping and administration is detailed in Table 8.

**Table 8. Animal grouping and administration**

| Group No. | Group | Drug type | Administration dose (mg/kg) | Drug concentration (mg/mL) | Route of administration | Administration volume (mL/200 g) | Animal Number |
|---|---|---|---|---|---|---|---|
| 1 | Control | DMSO:20 % solutol (5%:95%) | -- | -- | Intragastric | 1 | 7 |
| 2 | Model | DMSO:20 % solutol (5%:95%) | -- | -- | Intragastric | 1 | 7 |
| 3 | Pim-T | Pim-T | 0.70 | 0.140 | Intragastric | 1 | 7 |
| 4 | Formula I-0.22 | Formula I | 0.22 | 0.044 | Intragastric | 1 | 7 |
| 5 | Formula I-0.66 | Formula I | 0.66 | 0.133 | Intragastric | 1 | 7 |
| 6 | Formula I-2.0 | Formula I | 2.00 | 0.400 | Intragastric | 1 | 7 |

Note: The doses of Pim-T (pimavanserin tartrate) and the compound of Formula I were calculated based on the free base.

### 3. Experimental procedures

The rats were fasted starting at 5:00 PM the day before the experiment. On the day of the experiment, the animals were acclimatized in the testing laboratory for at least 1 h. Rats in each group were subjected to single intragastric administration at the corresponding dose listed in Table 3. One hour after administration, all groups except for the Control group were intraperitoneally injected with DOI at 2.5 mg/kg, while the Control group received an equal volume of normal saline. The behavior of the rats was observed immediately after the injection. The observations were conducted in a randomized, double-blind manner to eliminate interference of human factors. The number of head twitches in the rats was recorded within 1.0-1.5 h after the administration.

Experimental data were expressed as mean ± standard deviation (Mean ± SEM). One-way analysis of variance (ANOVA) was performed using the SPSS statistics 20.0 software to compare differences between the groups at each time point. All tests were bilateral tests, and P < 0.05 indicates being statistically significant.

### 4. Data analysis

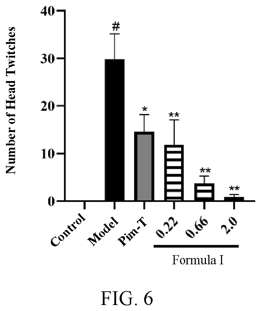

Compared to the Control group, the number of head twitches in the rats in the model group was significantly increased (P < 0.05). Compared to the model group, the number of head twitches in the rats in the Pim-T 0.7 mg/kg group was significantly reduced (P < 0.05), and the number of head twitches in the rats in the compound of Formula I 0.22 mg/kg, 0.66 mg/kg, and 2.0 mg/kg groups was extremely significantly reduced. (*P* < 0.01). At equimolar doses, the number of head twitches in the rats in the compound of Formula I 0.66 mg/kg group was lower than that in the Pim-T 0.7 mg/kg group. The specific results are shown in Table 9 and FIG. 6.

**Table 9. Number of head twitches in SD rats 1.0-1.5 h after single intragastric administration**

| Group No. | Group | Number of head twitches |
|---|---|---|
| 1 | Control | 0 |
| 2 | Model | 29.9 ± 5.3 ^{#} |
| 3 | Pim-T | 14.6 ± 3.6 * |
| 4 | Formula I-0.22 | 11.9 ± 5.1 ** |
| 5 | Formula I-0.66 | 3.7 ± 1.6 ** |
| 6 | Formula I-2.0 | 0.9 ± 0.6 ** |

| | | |
|---|---|---|
| Note: ^{#} indicates *P* < 0.05, as compared to the Control group; * indicates *P* < 0.05 and ** indicates *P* < 0.01, as compared to the Model group. | | |

The results show that: 1.0-1.5 h after single intragastric administration in SD rats, the compound of Formula I was able to significantly reduce the number of head twitches in the rats within the dose range of 0.22-2.0 mg/kg, with the effective dose being 0.22 mg/kg. At equimolar doses, the pharmacodynamic effect of the compound of Formula I was better than that of Pim-T.

### Test Example 5. Evaluation of In Vivo Tissue Distribution

### 1. Experimental protocol

SD rats (200-250 g) were subjected to single intragastric administration of the compound of Formula I at a dose of 11 mg/kg. Plasma and brain, heart, liver and lung tissue samples were collected at 0.25 h, 1 h, and 6 h after administration, with 3 animals used for each time point.

### 2. Experimental procedures

The rats were fasted starting at approximately 6:00 PM the day before the experiment, with free access to water during the fasting period. On the day of the experiment, the rats were weighed and randomly divided into 3 groups according to the body weight, with one group of animals assigned to one time point. The compound was dissolved in DMSO:20% solutol (5%:95%). The rats were subjected to single intragastric administration of the compound of Formula I at a dose of 11 mg/kg, with an administration volume of 5 mL/kg and a drug solution concentration of 2.2 mg/mL. At each sampling time point, the animals were anesthetized with ether, and about 1 mL of blood was collected from the heart and then placed in a heparinized EP tube. The blood sample was centrifuged at 10000 rpm for 10 min to separate the plasma. After cardiac perfusion to remove blood, the brain, heart, liver, and lung tissues were collected. The blood stains on the tissues were blotted dry with filter paper, and the tissues were separately weighed, then wrapped in weighing paper, and stored at -80 °C for further assay.

### 3. Sample assay and data analysis

Plasma samples and tissue homogenates (each tissue was homogenized in water at a weight-to-volume ratio of 1:4) were pretreated and then analyzed by LC-MS/MS to determine the concentrations of the test substance in plasma and tissues. Experimental data were expressed as mean ± standard deviation (Mean ± SD), and the specific results are shown in Table 10.

**Table 10. Plasma and tissue concentrations after single intragastric administration of the compound of Formula I in SD rats**

| Plasma/tissue | Hour | Mean ± SD | CV (%) | Ratio to plasma |
|---|---|---|---|---|
| Plasma | 0.25 | 338 ± 70.5 | 20.9 | / |
| | 1 | 247 ± 90.4 | 36.6 | / |
| | 6 | 274 ± 62.6 | 22.8 | / |
| Brain | 0.25 | 3216 ± 1428 | 44.4 | 9.51 |
| | 1 | 2376 ± 872 | 36.7 | 9.63 |
| | 6 | 2871 ± 624 | 21.7 | 10.5 |
| Heart | 0.25 | 1544 ± 382 | 24.7 | 4.57 |
| | 1 | 963 ± 387 | 40.2 | 3.90 |
| | 6 | 1137 + 270 | 23.8 | 4.16 |
| Liver | 0.25 | 11787 ± 1664 | 14.1 | 34.9 |
| | 1 | 4260 ± 1255 | 29.5 | 17.3 |
| | 6 | 5243 ± 1477 | 28.2 | 19.2 |
| Lung | 0.25 | 9440 ± 3776 | 40.0 | 27.9 |
| | 1 | 8067 ± 849 | 10.5 | 32.7 |
| | 6 | 8200 ± 1601 | 19.5 | 30.0 |

The results show that: at 0.25 h, 1 h, and 6 h after single intragastric administration in SD rats, the concentrations of the compound of Formula I in tissues were higher than that in plasma, indicating that the compound of Formula I had good permeability; particularly, its distribution in brain tissue was relatively high, with a brain-to-plasma ratio of 9.51-10.5, and the concentration in the brain remained stable between 0.25 h and 6 h.

### Test Example 6. Stability Study of Crystal Form E

A stability study of the Crystal Form E of the fumarate of the compound of Formula I was conducted under high temperature (60 °C) and light exposure (25 °C/4500 Lux) conditions. Samples were taken for XRPD characterization and HPLC analysis at 7 and 15 days. The results are shown in Table 11. The XRPD and HPLC results show that the Crystal Form E did not transition to another crystal form under high temperature and light exposure conditions for 15 days, and the purity did not change significantly.

**Table 11. Results of stability study of Crystal Form E of fumarate of the compound of Formula I**

| 0-day crystal form (purity/%) | Conditions | 7 days | | 15 days | |
|---|---|---|---|---|---|
| | | XRPD result | Purity/% | XRPD result | Purity/% |
| Crystal E (Purity: 99.96%) | High temperature 60 °C | No change in XRPD | 99.95 | No change in XRPD | 99.60 |
| | Light exposure 25 °C/4500 Lux | No change in XRPD | 99.97 | No change in XRPD | 99.97 |

### Test Example 7. Biological Medium and Water Solubility Test

The Crystal Form E of the fumarate of the compound of Formula I was subjected to dynamic solubility assays in 3 biological media (FaSSIF, FeSSIF, and FaSSGF) and water. The preparation process of biological media is shown in Table 12. The crystal form E was added to each of the biological media and water, and the mixture was shaken at a constant temperature of 37 °C for 24 h. Sampling was performed at 0.5 h, 2 h, and 24 h. The sampled solutions were filtered through a 0.22 µm aqueous filter membrane, and some samples with higher concentrations were properly diluted with a diluent. The signal peak areas of the solutions were measured by HPLC. Finally, the concentrations of the compound in the solutions were calculated according to the peak areas, HPLC standard curves of starting materials, and dilution factors. In addition, the 24-h supernatant was taken and tested for pH value, and the remaining solids were subjected to XRPD assay. The test results are shown in Table 13.

**Table 12. Preparation process of biological media**

| Biological medium | Preparation process |
|---|---|
| FaSSIF | 2.0825 g of FaSSIF concentrated solution was weighed out, added to a 50 mL volumetric flask, and diluted with a proper amount of pure water, and then 112.1 mg of FaSSIF powder was added. The volume was brought to 50 mL, and the mixture was mixed well (pH 6.5). |
| FeSSIF | 4.0731 g of FeSSIF concentrated solution was weighed out, added to a 50 mL volumetric flask, and diluted with a proper amount of pure water, and then 559.8 mg of FeSSIF powder was added. The volume was brought to 50 mL, and the mixture was mixed well (pH 5.0). |
| FaSSGF | 3.6783 g of FaSSGF concentrated solution was weighed out, added to a 100 mL volumetric flask, and diluted with a proper amount of pure water, and then 6.0 mg of FaSSGF powder was added. The volume was brought to 100 mL, and the mixture was mixed well (pH 1.6). |

**Table 13. Dynamic solubility test in biological media and water**

| Crystal form | Medium | pH | Sample amount (mg) | Volume (mL) | Solubility (mg/mL) | | | Final pH | XRPD of remaining solids |
|---|---|---|---|---|---|---|---|---|---|
| | | | | | 0.5 h | 2 h | 24 h | | |
| | FaSSIF | 6.5 | 39.6 | 4.0 | >9.63 | >9.83 | >9.68 | 4.2 | No remaining solids |
| Crystal E | FeSSIF | 5.0 | 40.0 | 4.0 | >9.60 | >9.80 | >9.58 | 4.7 | No remaining solids |
| | FaSSGF | 1.6 | 39.8 | 4.0 | >9.01 | >9.29 | >9.22 | 1.9 | No remaining solids |
| | Water | / | 40.0 | 4.0 | >9.03 | >9.37 | >9.15 | 3.6 | No remaining solids |

The results show that the solubility of the Crystal Form E in biological media and water was greater than 9 mg/mL at 24 h, and there were no remaining solids in FaSSIF, FeSSIF, FaSSGF, and water.

## Claims

1. A pharmaceutically acceptable salt of a compound of Formula I, wherein the pharmaceutically acceptable salt is selected from the group consisting of hydrochloride, sulfate, p-toluenesulfonate, maleate, phosphate, dihydroxybenzoate, mandelate, malate, succinate, acetate, hexanoate, palmitate, pamoate, citrate, L-tartrate, or fumarate, and is preferably fumarate,

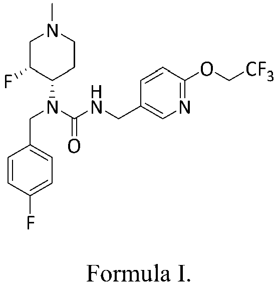

2. A Crystal Form A of the fumarate of the compound of Formula I according to claim 1, wherein the Crystal Form A has, in an X-ray powder diffraction pattern using Cu-Kα radiation, characteristic diffraction peaks at the following 2θ angles: 7.564±0.2°, 18.942±0.2°, 22.787±0.2°, and 26.629±0.2°; preferably, the Crystal Form A has characteristic diffraction peaks at the following 2θ angles: 7.564±0.2°, 11.335±0.2°, 15.212±0.2°, 18.942±0.2°, 22.787±0.2°, 24.571±0.2°, and 26.629±0.2°; more preferably, the X-ray powder diffraction pattern using Cu-Kα radiation is as shown in FIG. 1-1.

3. The Crystal Form A according to claim 2, wherein the Crystal Form A is an anhydrous crystal form.

4. A Crystal Form B of the fumarate of the compound of Formula I according to claim 1, wherein the Crystal Form B has, in an X-ray powder diffraction pattern using Cu-Kα radiation, characteristic diffraction peaks at the following 20 angles: 3.665±0.2°, 7.299±0.2°, 18.225±0.2°, 21.887±0.2°, 25.564±0.2°, and 29.164±0.2°; preferably, the Crystal Form B has characteristic diffraction peaks at the following 20 angles: 3.665±0.2°, 7.299±0.2°, 10.921±0.2°, 18.225±0.2°, 19.628±0.2°, 21.887±0.2°, 25.564±0.2°, 25.949±0.2°, and 29.164±0.2°; more preferably, the Crystal Form B has characteristic diffraction peaks at the following 20 angles: 3.665±0.2°, 7.299±0.2°, 10.921±0.2°, 11.923±0.2°, 18.225±0.2°, 19.628±0.2°, 21.061±0.2°, 21.887±0.2°, 25.564±0.2°, 25.949±0.2°, and 29.164±0.2°; most preferably, the X-ray powder diffraction pattern using Cu-Kα radiation is as shown in FIG. 2-1.

5. The Crystal Form B according to claim 4, wherein the Crystal Form B is an anhydrous crystal form.

6. A Crystal Form C of the fumarate of the compound of Formula I according to claim 1, wherein the Crystal Form C has, in an X-ray powder diffraction pattern using Cu-Kα radiation, characteristic diffraction peaks at the following 20 angles: 24.435±0.2°, 28.829±0.2°, and 38.312±0.2°; preferably, the Crystal Form C has characteristic diffraction peaks at the following 20 angles: 21.245±0.2°, 24.435±0.2°, 28.829±0.2°, 38.312±0.2°, and 39.979±0.2°; more preferably, the X-ray powder diffraction pattern using Cu-Kα radiation is as shown in FIG. 3-1.

7. A crystal form D of the fumarate of the compound of Formula I according to claim 1, wherein the Crystal Form D has, in an X-ray powder diffraction pattern using Cu-Kα radiation, characteristic diffraction peaks at the following 20 angles: 7.160±0.2°, 18.004±0.2°, 21.436±0.2°, and 25.082±0.2°; preferably, the Crystal Form D has characteristic diffraction peaks at the following 20 angles: 3.537±0.2°, 7.160±0.2°, 15.745±0.2°, 18.004±0.2°, 18.893±0.2°, 19.269±0.2°, 21.436±0.2°, and 25.082±0.2°; more preferably, the Crystal Form D has characteristic diffraction peaks at the following 20 angles: 3.537±0.2°, 7.160±0.2°, 10.700±0.2°, 15.745±0.2°, 18.004±0.2°, 18.893±0.2°, 19.269±0.2°, 20.965±0.2°, 21.436±0.2°, 25.082±0.2°, and 28.762±0.2°; most preferably, the X-ray powder diffraction pattern using Cu-Kα radiation is as shown in FIG. 4.

8. A Crystal Form E of the fumarate of the compound of Formula I according to claim 1, wherein the Crystal Form E has, in an X-ray powder diffraction pattern using Cu-Kα radiation, characteristic diffraction peaks at the following 2θ angles: 7.570±0.2°, 15.357±0.2°, 18.889±0.2°, 21.436±0.2°, 23.168±0.2°, and 27.084±0.2°; preferably, the Crystal Form E has characteristic diffraction peaks at the following 20 angles: 3.769±0.2°, 7.570±0.2°, 15.357±0.2°, 16.847±0.2°, 18.410±0.2°, 18.889±0.2°, 19.210±0.2°, 20.780±0.2°, 21.436±0.2°, 23.168±0.2°, 25.411±0.2°, 26.639±0.2°, 27.084±0.2°, 28.770±0.2°, 31.065±0.2°, and 32.507±0.2°; more preferably, the X-ray powder diffraction pattern using Cu-Kα radiation is as shown in FIG. 5-1.

9. The Crystal Form E according to claim 8, wherein the Crystal Form E is an anhydrous crystal form.

10. A pharmaceutical composition, comprising the pharmaceutically acceptable salt according to claim 1 or the crystal form according to any one of claims 2-9, and a pharmaceutically acceptable carrier.

11. Use of the pharmaceutically acceptable salt according to claim 1, the crystal form according to any one of claims 2-9, or the pharmaceutical composition according to claim 10 in the preparation of a medicament for treating a 5-HT2A receptor-related disease.

12. The use according to claim 11, wherein the 5-HT2A receptor-related disease comprises: schizophrenia, psychosis, schizoaffective disorder, manic disorder, psychotic depression, affective disorder, dementia, anxiety disorder, sleep disorder, appetite disorder, bipolar disorder, psychosis secondary to hypertension, migraine, hypertension, thrombosis, vasospasm, ischemia, motor tics, depression, major depressive disorder, anxiety, sleep disturbance and appetite disturbance, non-motor symptoms caused by Parkinson's disease (comprising delusion, hallucination, depression, anxiety, cognitive disorder, or sleep disorder), Alzheimer's disease psychosis (comprising delusion and hallucination), negative symptoms of schizophrenia, Parkinson's disease, Huntington's chorea, Alzheimer's disease, spinocerebellar atrophy, Tourette's syndrome, Friedreich's ataxia, Machado-Joseph disease, Lewy body dementia, movement disorder, dystonia, myoclonus, tremor, or progressive supranuclear palsy and frontotemporal dementia.
